# EUROPEAN PATENT APPLICATION

(11) **EP 1 547 586 A1**
(43) Date of publication of application: **29.06.2005**
(21) Application number: 03794189.5
(22) Date of filing: 02.09.2003
(51) Int. Cl.: A61K 31/18, A61P 13/02, A61P 29/00, A61P 43/00

(54) **HYPOGASTRIC AND/OR PERINEAL PAIN-RELIEVING AGENT**

(30) Priority: 03.09.2002 JP 2002258019
(71) Applicant: YAMANOUCHI PHARMACEUTICAL CO. LTD., Tokyo 103-8411 (JP)
(72) Inventor: FURUDATE, Naomichi, c/o Yamanouchi Pharm. Co. Ltd., Tokyo 174-8612 (JP); SHIMOYAMA, Mitsuru, c/o Yamanouchi Pharm. Co. Ltd., Tokyo 174-8612 (JP)
(74) Representative: HOFFMANN EITLE
(86) International application number: PCT/JP2003/011211
(87) International publication number: WO 2004/022045

(57) **Abstract**

A pharmaceutical composition for improving chronic pelvic cavity pain syndrome due to urinary dysfunction where said composition contains tamsulosin or pharmaceutically acceptable salts thereof.

## Description

### Technical Field of the Invention

The present invention relates to a pharmaceutical agent and, more particularly, it relates to a therapeutic agent for improving chronic pelvic cavity pain syndrome due to urinary dysfunction.

### Background Art

In patients with obstructive urinary dysfunctions including prostatic hyperplasia and lower uropathy, it is known that itchiness, discomfort and burning sensation occur in the urethra. On the other hand, apart from these urethra-related symptoms, discomfort and pain may occur at the hypogastric/perineal region. It is thought that the bladder is loaded with pooled urine, and this visceral loading is transmitted to other organs as stimuli, which are then expressed as pains at the hypogastric/perineal region or about the lower back region. Recently, the diagnostic name of chronic pelvic cavity pain syndrome is used for these hypogastric/perineal pain and lower backache due to decreased urine production (1995 NIH Workshop on Chronic Prostatitis/Urology, 60(1), 74-77, 2002). A drug capable of improving/mitigating this chronic palvic cavity pain syndrome due to urinary dysfunction is still not reported, and the development of such a drug is eagerly anticipated.

### Disclosure of the Invention

In the situation described above, the present inventor found that tamsulosin or its salt is effective in the improvement of chronic pelvic cavity pain syndrome due to urinary dysfunction.

Thus, the present invention relates to a pharmaceutical composition for improving chronic pelvic cavity pain syndrome due to urinary dysfunction where said composition contains tamsulosin or pharmaceutically acceptable salts thereof. In other words, the present invention relates to a pharmaceutical composition for improving hypogastric/perineal pain due to a state of decreased urine production where said composition contains tamsulosin or pharmaceutically acceptable salts thereof.

In addition, the present invention relates to an use of tamsulosin or its pharmaceutically acceptable salt thereof for the manufacture of a therapeutic agent for improving chronic pelvic cavity pain syndrome due to urinary dysfunction, and also relates to an use of tamsulosin or a pharmaceutically acceptable salt thereof for the manufacture of a therapeutic agent for improving hypogastric/perineal pain due to a state of decreased urine production.
Moreover, the present invention relates to a method for the therapy of chronic pelvic cavity pain syndrome due to urinary dysfunction, where said method includes the administration of effective therapeutic doses of tamsulosin or a pharmaceutically acceptable salt thereof to patients, and also relates to a method for the therapy of hypogastric/perineal pain due to a state of decreased urine production, where said method includes the administration of effective therapeutic doses of tamsulosin or a pharmaceutically acceptable salt thereof to patients.
The chemical name of tamsulosin is (R)(-)-5-[2-[[2-(o-ethoxyphenoxy)ethyl]amino]propyl]-2-met hoxybenzenesulfonamide, and represented by the structural formula shown below. Tamsulosin together with a pharmaceutically acceptable salt thereof were first disclosed in Japanese Published Unexamined Application No. 56-110665.

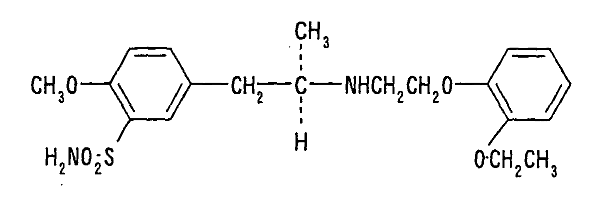

Tamsulosin and its salts are known to block the adrenergic α _{1A} receptor. In particular, tamsulosin hydrochloride possesses an antagonistic action on urethral and prostatic α ₁ receptors and is used widely as a drug capable of lowering the prostatic pressure of the urethral pressure profile and consequently ameliorating the urinary dysfunction associated with prostatic hyperplasia. The effectiveness of tamsulosin hydrochloride on dysuria and lower urinary tract symptoms due to neurogenic bladder (urinary dysfunction associated with the functional obstruction of the lower urinary tract but without accompanying obvious organic impairment or neurological abnormality in lower urinary tract) has also been confirmed (WO00/00187, WO01/10436).

While the report confirming the efficacy on hypogastric/perineal pains or lower backache due to urinary dysfunction was not available, the present inventor clinically confirmed for the first time that tamsulosin hydrochloride is effective in the improvement of chronic pelvic cavity pain syndrome (hypogastric/perineal pain or lower backache due to a state of decreased urine production).

The present invention is explained in greater details below.

In the present invention, the chronic pelvic cavity pain syndrome due to urinary dysfunction is to mean the condition in which a state of decreased urine production is perceived at the hypogastric/perineal region or lower back region as pain, discomfort, burning sensation, itching sensation, aching, heat sensation, dull pain, irritating sensation, sense of discomfort, abnormal sensation, feeling of oppression, feeling of colic, feeling of instability, feeling of ataxia, feeling of convulsion, beating sensation, feeling of numbness, or fatigue. The term, urinary dysfunction, is to include the urinary dysfunction associated with organic obstruction of the urethra such as prostatic hyperplasia, the urinary dysfunction associated with abnormalities in urination controlling nerves such as neurogenic bladder, and lower urinary tract symptoms (1995 NIH Workshop on Chronic Prostatitis/Urology, 60(1), 74-77, 2002).

The term, a therapeutic agent for improving chronic pelvic cavity pain syndrome due to urinary dysfunction, means a drug to improve/mitigate the symptoms shown above.
Tamsulosin and a pharmaceutically acceptable salt thereof can be manufactured and is readily available according to the manufacturing method described in Japanese Published Unexamined Applications No. 56-110665 and No. 62-114952, or equivalent methods.
Tamsulosin can form pharmaceutically acceptable salts with a wide range of inorganic and organic acids or bases. Such salts are also included in the present invention. For example, tamsulosin forms salts with inorganic acids such as hydrochloric acid, sulfuric acid and phosphoric acid, with organic acids such as fumaric acid, malic acid, citric acid and succinic acid, with alkaline metals such as sodium and potassium, and with alkaline earth metals such as calcium and magnesium. The most preferable salt in the present invention is the hydrochloride.

The drug of the present invention can be prepared according to conventional methods as an oral solid formulation, oral liquid formulation, or a formulation for injection, using the organic or inorganic carriers, excipients and other additives, that are suitable for oral or parenteral administration. The oral solid formulation is preferable, because patients themselves can take it easily, and store and carry it conveniently. Concretely, tablets, power, granules, capsules and pills are included in the oral solid formulation.

In these solid formulations, the active substance is mixed with at least one inactive diluent such as, for example, lactose, mannitol, glucose, fine crystal cellulose, starch, polyvinylpyrrolidone, and magnesium aluminometasilicate. The composition may contain in the conventional manner the additives other than inactive diluents, such as, for example, binders such as hydroxypropyl cellulose and hydroxypropyl methylcellulose, lubricants such as magnesium stearate, calcium stearate, polyethylene glycol, starch, and talc, disintegrating agents such as cellulose calcium glycolate, stabilizers such as lactose, solubilizing agents such as glutamic acid or aspartic acid, elasticizers such as Tween 80 and triacetin, and colorants such as titan oxide and iron sesquioxide. The tablets and pills may be sugarcoated using sucrose, gelatin, agar, pectin, hydroxypropyl cellulose and hydroxypropyl methylcellulose phthalate, if needed, or filmed with gastric or enteric substances.

The most preferable formulation in the present invention is a sustained release preparation. The sustained release formulation may be prepared as tablets, granules, fine granules and capsules by known methods. These sustained release formulations may be obtained by coating tablets, granules, fine granules and capsules with, for example, oils and fats, fatty acid esters of polyglycerin, and hydroxypropyl cellulose in the conventional manner.

The sustained release formulation disclosed in Japanese Published Unexamined Application No. 62-9 is preferable in particular.
Namely, each unit formulation is either a capsule or a tablet, prepared respectively by capsuling or tableting granules obtained by granulating a mixture of an active ingredient and a unit forming substance in an amount such that 50 % or more by weight based on the weight of the unit is said unit forming substance, after adding a release controlling agent to said mixture. Crystal cellulose is preferable as the unit forming substance. As the elution suppressor, water-insoluble high molecular substances such as, for example, acrylic acid polymers and copolymers, or cellulose derivatives are used. It is appropriate to use these substances in the form of aqueous suspension, aqueous emulsion, and water-containing organic solvent solution. For example, Eudragit L30D-55 (methacrylic acid copolymer LD), Eudragit E30D (ethylacrylate/methyl methacrylate copolymer emulsion), and Aquacoat ECD-30 (ethylcellulose aqueous suspension) are available commercially. These elution suppressors may be used as they are, or after diluting with water, if needed.

In addition, the persistent oral absorption-type sustained release formulation capable of releasing a drug nicely in not only upper but also lower digestive tracts and consequently sustaining the constant drug release for such a long time as 12 to 24 hr after oral take is also preferable.

For example, the persistent oral absorption-type sustained release formulation disclosed in WO94/06414 can release a drug even in the colon where little water is available, because this formulation absorbs water into the inside of the formulation while being retained in upper digestive tracts and the formulation gelatinized nearly completely is transferred to lower digestive tracts. Concretely, this is the hydro-gel sustained release tablet with a gelatinization ratio of ≧ 70% and < 100%, and comprising of (1) a drug, (2) an additive for making water penetrate into the tablet inside of the tablet which occupies 5 to ≧ 80% (w/w) of the total weight of a tablet and dissolves ≦ 5 ml water/g, and (3) the polymer which is ≧ 70 mg/tablet, 10 to 95% (w/w) of the total weight of a tablet and ≧ 2,000,000 in mean molecular weight, and forms hydro-gel with a viscosity of ≧ 1000 cps in 1% aqueous solution at 25°C. As additives for making water penetrate into the inside of the tablet, the following substances are used: Polyethylene glycol, polyvinylpyrrolidone, D-sorbitol, xylitol, white sucrose, anhydrous maltose, D-fructose, dextran, glucose, polyoxyethylene polyoxypropylene glycol, sodium chloride, magnesium chloride, citric acid, tartaric acid, glycine, β -alanine, lysine chloride, and meglumine. As high-molecular substances forming hydro-gel, the following substances are used: Polyethylene oxide, hydroxypropyl methylcellulose, sodium carboxymethylcellulose, hydroxymethyl cellulose, and carboxyvinyl polymers. When polyethylene oxide is used as a hydro-gel-forming polymer, photostability may be achieved by adding yellow iron sesquioxide and/or red iron sesquioxide, as disclosed in WO01/10466.

The dose of tamsulosin or a pharmaceutically acceptable salt thereof is determined as appropriate for individual cases taking the administration route, symptoms, and the age and gender of administration subjects into consideration. Based on the study results to be described later, in the case of usual oral administration, the dose of the effective ingredient per an adult patient is 0.1 to 2.0 mg/day, and the most preferable dose is 0.25 to 1.5 mg/day. This is orally administered once daily after meal.

Although the drug of the present invention is effective enough in single administration, it may be co-administered simultaneously, or with an interval, with a cholinergic agonist, anti-choline drug, and other central nervous system drugs.

### Best Mode for Carrying out the Invention

The present invention is explained in more details below based on examples and study examples, but the present invention is not limited to these examples etc.

### Example 1.

5 g of tamsulosin hydrochloride and 470 g of crystalline cellulose were well mixed, 500 g of a mixture of 83.3 g of Eudragit L30D-55 (25 g as a solid) with water were added thereto and the mixture was granulated using a high-speed stirring granulator. The resulting particles were in a spherical shape having a particle size of 0.1-1.5 mm where most of them were 0.2-1.0 mm.

The resulting particles were mixed with talc and magnesium stearate and filled in capsules to prepare capsule preparations (containing 0.2 mg of tamsulosin hydrochloride in a capsule).

### Examples 2-6.

The same process as in Example 1 was conducted whereby the particles manufactured according to the formulations of Table 1 were made into capsule preparations.

**Table 1**

| (unit: grams) | | | |
|---|---|---|---|
| Example Number | Tamsulosin Hydrochloride (g) | Crystalline Cellulose (g) | Eudragit L30D-55 (Solid Content) (g) |
| 2 | 5 | 445 | 166.6 (50) |
| 3 | 5 | 395 | 333.3 (100) |
| 4 | 5 | 482.5 | 41.7 (12.5) |
| 5 | 2.5 | 472.5 | 83.3 (25) |
| 6 | 1.25 | 473.75 | 83.3 (25) |

### Example 7.

5 g of tamsulosin hydrochloride, 420 g of crystalline cellulose and 50 g of magnesium stearate were well mixed, a mixture of 83.3 g of Eudragit L30D-55 (25 g as a solid) with water were added thereto and the mixture was kneaded followed by granulating by a centrifugal fluid granulator. The resulting particles were in a spherical shape having a particle size of 0.1-1.5 mm where most of them were 0.2-1.0 mm.

The resulting particles were mixed with talc and magnesium stearate and filled in capsules to prepare capsule preparations (containing 0.2 mg of tamsulosin hydrochloride in a capsule).

### Examples 8-10.

The same process as in Example 7 was conducted whereby the particles manufactured according to the formulations of Table 2 were made into capsule preparations.

**Table 2**

| (unit: grams) | | | | |
|---|---|---|---|---|
| Example Number | Tamsulosin Hydrochloride | Crystalline Cellulose | Magnesium Stearate | Eudragit L30D-55 (Solid Content) |
| 8 | 5 | 460 | 10 | 83.3 (25) |
| 9 | 2.5 | 462.5 | 25 | 83.3 (25) |
| 10 | 5 | 445 | 10 | 83.3 (25) |

### Example 11.

80 g of hydrogenated castor oil was melted, 10 g of tamsulosin hydrochloride and 30 g of hydroxypropyl cellulose having a low degree of substitution were dispersed therein and the dispersion was made into fine particles by means of a spray condyling. The resulting fine particles (60 g) were well mixed with 440 g of crystalline cellulose, 500 g of water were added thereto and the mixture was granulated using a centrifugal fluid granulator.

The resulting particles were mixed with talc and magnesium stearate and filled in capsules to prepare capsule preparations.

### Example 12

| | |
|---|---|
| Tamsulosin hydrochloride | 0.2 (mg) |
| D-Sorbitol | 17.8 |
| Polyethylene oxide (POLYOX WSR N-60K) | 180 |
| Lubricant | 2 |

Tamsulosin hydrochloride, D-sorbitol and polyethylene oxide (POLYOX WSR N-60K) were granulated with wet granulation method using ethanol and dried. Dried product was mixed with a lubricant and tableted to obtain the persistent oral absorption-type sustained release formulation with a diameter of 8 mm and a weight of 200 mg/tablet.

### Example 13

The persistent oral absorption-type sustained release formulation with the prescription shown below was manufactured in a manner similar to Example 12.

| | 0.25mg Tablets | 0.5mg Tablets | 1.0mg Tablets |
|---|---|---|---|
| Tamsulosin-HCl | 0.25mg | 0.5mg | 1.0mg |
| Polyethylene glycol 8000 | 40 | 40 | 40 |
| Polyethylene oxide (POLYOX WSR 303) | 200 | 200 | 200 |
| Magnesium stearate | 1.2 | 1.2 | 1.2 |

### Test Example 1. Clinical Test (12 week study) to Patients Suffering from Lower Urinary Tract Symptoms

A clinical study was conducted in patients with lower urinary tract symptoms under the conditions shown below.
Subjects: Eighteen patients diagnosed to have urinary dysfunction without accompanying obvious organic or neurological abnormality in lower urinary tract.
Study drug and the method of administration: Until Week 4, capsules each containing 0.2 mg tamsulosin hydrochloride were orally administered, one capsule/time/day, after breakfast. In Week 4, the doses for later than Week 4 were determined by referring to the Table below, and the doses determined were orally administered once daily after breakfast.

### <Criterion of Dose after 4 weeks>

| | | |
|---|---|---|
| side effects | Changing Rate of Total score for subjective symptoms in 4 weeks | dose of after 4 weeks |
| yes | | 0.2mg/day |
| no | Changing Rate of Total score for subjective symptoms ≦ -25.0% | |
| | Changing Rate of Total score for subjective symptoms ≧ -24.9% | 0.4mg/day |

### Study period: 12 weeks

Observed items: Evaluations and measurements were made for the following items before and after the administration.

### (1) Total score for subjective symptoms

Questioning was done to the patients for the following items and the total score was obtained.

### <1> Feel of residual urine

"How often do you have a sensation of not emptying your bladder completely after you finished urinating?"
0: not at all
1: not so often
2: sometimes
3: about half the time
4: often
5: almost always

### <2> Urination within two hours

"How often do you have to urinate again less than two hours after you finished urinating?"
0: not at all
1: not so often
2: sometimes
3: about half the time
4: often
5: almost always

### <3> Break of urinary stream

"How often do you find you stopped and started again several times when you urinated?"
0: not at all
1: not so often
2: sometimes
3: about half the time
4: often
5: almost always

### <4> Urgency

"How often do you find it difficult to postpone urination?"
0: not at all
1: not so often
2: sometimes
3: about half the time
4: often
5: almost always

### <5> Force of urinary stream

"How often do you have a weak urinary stream?"
0: not at all
1: not so often
2: sometimes
3: about half the time
4: often
5: almost always

### <6> Straining upon urination

"How often do you have to push or strain to begin urination?"
0: not at all
1: not so often
2: sometimes
3: about half the time
4: often
5: almost always

### <7> Frequency of urination at night

"How many times do you most typically get up to urinate from the time you go to bed at night until the time you get up in the morning?"
0: none
1: one times
2: two times
3: three times
4: four times
5: five or more times

### (2) Pains

The pain scores shown below were evaluated before administration, and 4 and 12 weeks after administration.

### <1> Hypogastric/perineal pain

"How strong is hypogastric/perineal pain?"
0: No pain
1: Slightly painful
2: Painful
3: Quite painful
4: Very painful

### <2> Lower backache

"How strong is lower backache?"
0: No pain
1: Slightly painful
2: Painful
3: Quite painful
4: Very painful

Of 18 patients, 7 patients complained of hypogastric/perineal pain before administration, and the score was 1 (slightly painful) in any of them. In the evaluation in Weeks 4 and 12 of administration, 6 patients were improved to score 0 (no pain) and remaining 1 patient was unchanged (score 1, slightly painful).

Only 1 patient complained of lower backache before administration. Although the score of this patient was 1 (slightly painful), this was improved to score 0 (no pain) in the evaluation in Weeks 4 and 12 of administration.

These results are to confirm that tamsulosin hydrochloride is effective in the improvement of hypogastric/perineal pain or lower backache in patients with urinary dysfunction.

### Industrial Applicability

The present invention is expected to provide a clinically effective drug improving the chronic pelvic cavity pain syndrome due to urinary dysfunction.

## Claims

1. A pharmaceutical composition for improving chronic pelvic cavity pain syndrome due to urinary dysfunction where said composition contains tamsulosin or pharmaceutically acceptable salts thereof.

2. A pharmaceutical composition for improving hypogastric/perineal pain due to a state of decreased urine production where said composition contains tamsulosin or pharmaceutically acceptable salts thereof.

3. A pharmaceutical composition for improving chronic pelvic cavity pain syndrome due to urinary dysfunction according to Claim 1 where said composition contains tamsulosin hydrochloride.

4. Use of tamsulosin or a pharmaceutically acceptable salt thereof for the manufacture of a therapeutic agent for improving chronic pelvic cavity pain syndrome due to urinary dysfunction.

5. Use of tamsulosin or a pharmaceutically acceptable salt thereof for the manufacture of a therapeutic agent for improving hypogastric/perineal pain or lower backache due to a state of decreased urine production.

6. A method for the therapy of chronic pelvic cavity pain syndrome due to urinary dysfunction, where said method includes the administration of effective therapeutic doses of tamsulosin or a pharmaceutically acceptable salt thereof to patients.

7. A method for the therapy of hypogastric/perineal pain or lower backache due to a state of decreased urine production, where said method includes the administration of effective therapeutic doses of tamsulosin or a pharmaceutically acceptable salt thereof to patients.
